# FASCICULE DE BREVET EUROPEEN

(11) **EP 3 386 407 B1**
(45) Date de publication et mention de la délivrance du brevet: **08.06.2022**
(21) Numéro de dépôt: 16825802.8
(22) Date de dépôt: 06.12.2016
(51) Int. Cl.: A61B 18/12, A61B 90/50

(54) **APPAREIL ÉLECTRO-MEDICAL INCORPORANT UN DISPOSITIF DE DÉTECTION DU FONCTIONNEMENT D'OUTILS ÉLECTRO-CHIRURGICAUX**
ELEKTROMEDIZINISCHES GERÄT MIT EINER VORRICHTUNG ZUR ERKENNUNG DES BETRIEBS ELEKTROCHIRURGISCHER WERKZEUGE
ELECTRO-MEDICAL APPLIANCE INCORPORATING A DEVICE FOR DETECTING THE OPERATION OF ELECTRO-SURGICAL TOOLS

(30) Priorité: 09.12.2015 FR 1562085
(43) Date de publication de la demande: 17.10.2018
(73) Titulaire: Hemodia, 31670 Labège (FR)
(72) Inventeur: BOIREAU, Alan, 31750 Escalquens (FR); SANT, William, 31450 Montgiscard (FR); DIAS, Armando, 31670 Labege (FR)
(74) Mandataire: Plasseraud IP
(86) Numéro de dépôt international: PCT/FR2016/053223
(87) Numéro de publication internationale: WO 2017/098133

(56) Documents cités:
- EP-A1- 2 165 720
- US-A1- 2008 154 095
- US-A1- 2013 267 779
- US-A1- 2014 128 886

## Description

### Domaine Technique

La présente invention se rapporte de manière générale au domaine des appareils électro-médicaux et des outils chirurgicaux électro-médicaux.

Elle concerne plus particulièrement un appareil électro-médical, telle qu'une pompe d'arthroscopie, adaptée pour réagir à la détection du fonctionnement d'outils chirurgicaux électro-médicaux externes qui sont alimentés par câble électrique de manière indépendante de l'appareil.

### Arrière-plan Technologique

Des opérations chirurgicales mini-invasives telles que des opérations d'arthroscopie sont pratiquées, par exemple, pour effectuer des diagnostics, des prélèvements et des soins sur les articulations d'un patient.

A cet effet, les praticiens utilisent, généralement, un appareil électro-médical tel qu'une pompe d'arthroscopie qui irrigue la zone à traiter d'un liquide aseptique et aspire le liquide souillé par le sang, les bulles et les débris générés par les prélèvements ou les soins effectués, dans le but notamment d'assurer au chirurgien une vision claire de la zone à traiter.

Pour pratiquer les soins proprement dits, les chirurgiens peuvent utiliser différents outils électro-chirurgicaux tels que les « shavers » (sorte de fraise motorisée qui tourne de façon alternative et permet de « rabotter » une surface cartilagineuse pathologique), les bistouris électriques, les sondes radiofréquence (RF), etc. Ces différents instruments sont tous associés à un générateur alimentant électriquement, au travers d'un câble (blindé ou non), une pièce à main (porte-outil) sur laquelle est monté l'outil électro-chirurgical à utiliser.

Il est souhaitable de commander la mise en service de la pompe d'arthroscopie uniquement pendant les phases de fonctionnement des outils électro-chirurgicaux.

### Art Antérieur

Actuellement, cette synchronisation entre la mise en service de la pompe d'arthroscopie et le fonctionnement de l'outil électro-chirurgical est réalisée par l'intermédiaire de câbles, appelés communément « interfaces Shaver », qui sont connectés entre la pompe d'arthroscopie et le générateur d'alimentation de l'outil électro-chirurgical. La diversité des fabricants d'outils électro-chirurgicaux, chacun utilisant une solution différente, impose toutefois aux fabricants de pompes d'arthroscopie l'utilisation de connecteurs, et donc de câbles différents pour chaque fabricant d'outils afin de permettre de connecter la pompe aux générateurs d'alimentation de ces outils.

Dans un état de la technique reflété par la demande de brevet US 2014/0046249 et le brevet US 8,591,453 il est connu un dispositif de détection du fonctionnement d'un outil électro-chirurgical opérant par détection d'un courant électrique d'alimentation circulant dans le câble d'alimentation de l'outil électro-chirurgical lorsque ce dernier est en fonctionnement. La pompe d'arthroscopie n'est mise en service qu'en réponse à un signal de détection d'un tel courant, ce qui procure la synchronisation recherchée. Le dispositif de détection se présente sous la forme d'une pince ampèremétrique qu'il convient de fixer au câble d'alimentation du porte-outil. Un inconvénient de cet état de la technique réside dans le fait que la pince ampèremétrique doit elle-même être reliée à la pompe d'arthroscopie par un câble spécifique, ce qui augmente le coût et la complexité d'utilisation de cette appareil, et encombre le champ d'intervention du chirurgien.

### Résumé de l'Invention

L'invention vise à supprimer, ou du moins atténuer, tout ou partie des inconvénients de l'art antérieur précités.

A cet effet, un premier aspect de l'invention se rapporte à un appareil électro-médical comprenant un boîtier ayant avec un châssis et au moins une face de boîtier accessible par un utilisateur, et un dispositif interne d'activation conditionnelle de la mise en service d'au moins une fonction de l'appareil en réponse à la détection d'un courant électrique circulant dans un câble externe à l'appareil. L'appareil comprend en outre un système ayant :
- un dispositif de blocage maintien de câble agencé sur la face de boîtier, du côté externe à l'appareil, pour maintenir un câble externe à l'appareil au niveau de la face de boîtier dans une position de passage à proximité de la face de boîtier, et
- un dispositif de détection de courant, intégré dans l'appareil au niveau de la face de boîtier et du dispositif de blocage maintien de câble, pour détecter la circulation d'un courant dans un câble bloqué externe à l'appareil qui est maintenu dans le dispositif de blocage maintien de câble.

Par l'expression « câble externe à l'appareil », on entend un câble qui n'est pas couplé mécaniquement ni relié électriquement à l'appareil. Il s'agit dans l'exemple envisagé d'un câble d'alimentation électrique d'un autre appareil, distinct de l'appareil concerné, par exemple le générateur d'un outil électro-médical du type précité alors que l'appareil concerné peut être une pompe d'arthroscopie ayant son propre câble d'alimentation pour fonctionner de manière autonome.

La détection d'un courant circulant dans le câble externe à l'appareil qui est ainsi maintenu au niveau de la face de boîtier est une détection de type sans contact. Elle ne nécessite donc pas de dénuder le câble, ni de dispositif de raccordement du type d'une fiche ou d'un connecteur électrique.

Dans un mode de réalisation le dispositif de détection de courant peut comprendre une carte électronique avec une antenne associée à un circuit résonnant pour capter un signal correspondant à un bruit électronique à large bande spectrale généré par la circulation dans le câble d'un courant d'alimentation d'un outil chirurgical électro-médical, et à un circuit d'amplification et de traitement du signal capté pour générer un signal d'activation conditionnelle de la mise en service de la fonction de l'appareil.

La carte électronique peut par exemple être fixée au châssis à l'intérieur du boîtier de l'appareil par un étrier de fixation de telle manière qu'elle s'étend parallèlement à la face de boîtier. Dans un exemple non limitatif, le dispositif de maintien de câble peut alors comprendre une glissière avec une partie mobile agencée pour coulisser entre les branches de l'étrier de fixation parallèlement à la face de boîtier, à l'intérieur du boîtier.

Dans un exemple de réalisation la partie mobile de la glissière peut présenter une rainure longitudinale s'étendant suivant la direction de déplacement de la partie mobile de la glissière. Dans un exemple non limitatif, la glissière peut alors comprendre une partie dormante fixée au châssis.

Le dispositif de maintien de câble peut comprendre un bouton de préhension accessible par un utilisateur depuis l'extérieur de l'appareil et couplé mécaniquement à la partie mobile de la glissière à l'intérieur du boîtier, à travers la face de boîtier. Dans un exemple non limitatif, le dispositif de maintien peut alors comprendre, en outre, des moyens de rappel élastique pour ramener le bouton de préhension dans une position de repos lorsque l'utilisateur relâche le bouton de préhension après avoir exercé une action tendant à le déplacer par rapport à ladite position de repos.

Dans un mode de réalisation, le bouton de préhension peut comprendre une échancrure du côté de la position de repos du bouton si l'on considère la course dudit bouton entre deux positions extrêmes résultant des actions contraires de l'utilisateur et des moyens de rappel élastiques. Cette échancrure est prévue pour recevoir le câble externe à l'appareil.

Dans un autre mode de réalisation, en outre, la face de boîtier peut présenter un plan de contact en regard de l'échancrure du bouton de préhension, le diamètre de l'échancrure et l'espace entre le fond de l'échancrure et le plan de contact étant tels qu'un câble externe à l'appareil peut être amené à reposer dans l'échancrure lorsque l'utilisateur exerce une action sur le bouton tendant à la déplacer par rapport à sa position de repos et être pincé contre la paroi de contact lorsque l'utilisateur relâche le bouton et que le bouton, en conséquence, revient dans sa position de repos sous l'effet des moyens de rappel élastiques.

Dans un autre mode de réalisation, la face de boîtier peut comprendre en outre un blindage de protection électromagnétique de la carte électronique du côté de la face de boîtier, au niveau de la carte électronique.

Le système peut aussi comprendre, en outre, un capteur à effet Hall pour détecter la présence d'un câble bloqué dans le dispositif de maintien de câble.

Comme on l'aura compris, l'appareil peut être une pompe d'arthroscopie dans laquelle la fonction mise en service en réponse à la détection d'un courant électrique circulant dans le câble externe à l'appareil comprend l'irrigation par un fluide propre et l'aspiration de fluides souillés au niveau d'un site d'intervention chirurgicale pratiquée à l'aide d'outils chirurgicaux électro-médicaux alimentés électriquement par le câble externe à l'appareil.

L'invention procure ainsi une interface de détection simple, directement intégrée à l'appareil électro-médical, par exemple une pompe d'arthroscopie, permettant de synchroniser le fonctionnement de la pompe d'arthroscopie avec la mise en fonctionnement d'un outil chirurgical alimenté électriquement par un câble, sans exclusivité de marque ou de type d'outil électro-chirurgical.

Avantageusement, le système médical formé du dispositif de blocage de câble et du dispositif de détection de courant décrits sont utilisables dans toutes les spécialités chirurgicales nécessitant l'utilisation d'une pompe d'aspiration/irrigation des fluides et d'outils chirurgicaux électro-médicaux alimentés par câble.

Le système médical est adapté à tous types d'outils chirurgicaux électro-médicaux alimentés par câbles (de différents diamètres), blindés ou non, en particulier les Shavers et bistouris électriques, et électrodes radiofréquence.

Le système médical est adapté universellement à tout système Shaver sans distinction de marque ou de modèle.

La carte électronique est directement intégrée à la pompe d'arthroscopie, de préférence en face avant (façade) de celle-ci.

La carte électronique permet de détecter le bruit électronique large bande émis au travers du câble d'alimentation de l'outil électro-chirurgical lors du fonctionnement de celui-ci.

La carte électronique peut être installée dans un environnement blindé pour améliorer l'immunité aux parasites électromagnétiques.

Le dispositif de blocage de câble peut comprendre un système de fixation composé d'un étrier intégré à la pompe, d'un ressort et d'un bouton de préhension accessible de préférence en face avant de la pompe pour fixer tout câble électrique de diamètre indifférent (dans la mesure permise par la course du bouton de préhension et par le diamètre de l'échancrure qui peuvent être choisis en fonctions des spécificités des applications envisagées pour l'appareil), à proximité de l'antenne de la carte électronique.

Le système médical peut être muni d'un système à effet Hall pour détecter la présence d'un câble bloqué dans le dispositif de maintien de câble et ainsi contribuer à la réduction du risque de fausses détections, par exemple en permettant d'identifier comme une fausse détection une détaction réalisée alors qu'aucun câble n'est présent dans le système.

### Brève Description des Dessins

D'autres caractéristiques et avantages de l'invention apparaîtront encore à la lecture de la description qui va suivre. Celle-ci est purement illustrative et doit être lue en regard des dessins annexés sur lesquels :
- la Figure 1, est un schéma fonctionnel illustrant la cinématique du dispositif de blocage de câble selon des modes de réalisation de l'invention ;
- la Figure 2 est un schéma montrant un premier mode de réalisation et d'agencement des moyens de rappel élastique du bouton de la Figure 1 ;
- la Figure 3 est un schéma montrant un second mode de réalisation et d'agencement des moyens de rappel élastique du bouton de la Figure 1 ;
- la Figure 4 est une vue en trois dimensions du système médical selon des modes de réalisation ;
- la Figure 5 est une autre vue en trois dimensions du système de la Figure 4, d'un autre côté, avec le système fixé sur le châssis du boîtier de la pompe d'arthroscopie ;
- la Figure 6 est une vue en coupe latérale du système selon les Figures 4 et 5 ; et,
- la Figure 7 est un schéma d'un autre mode de réalisation du dispositif de blocage de câble, selon une variante,
- la Figure 8 représente une vue d'ensemble d'un système complet incluant un appareil électro-médical et un outil chirurgical.

### Description détaillée de modes de réalisation

Des modes de réalisation de l'invention vont être décrits ci-après, dans le contexte d'un appareil électro-médical qui est par exemple une pompe d'arthroscopie. Cet exemple n'est pas limitatif. L'invention peut en effet s'appliquer à tout type d'appareil électro-médical dans toutes les spécialités chirurgicales nécessitant l'utilisation d'un équipement tel qu'une pompe d'aspiration/irrigation de fluides, qui doit être mis en service en synchronisme avec le fonctionnement d'outils chirurgicaux électro-médicaux externes à l'appareil.

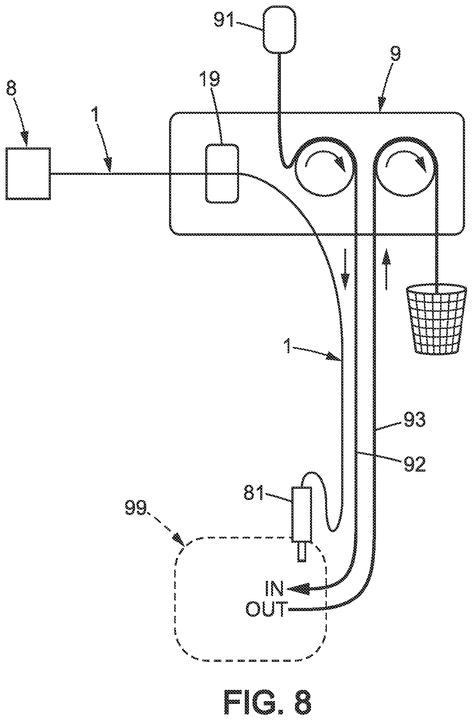

Dans l'exemple d'un système type représenté la figure 8, l'appareil électro-médical est une pompe d'arthroscopie **9** qui comprend deux pompes péristaltiques, pour d'une part amener (par la conduite 92) du liquide physiologique stérilisé depuis un réservoir **91** jusqu'à un site clinique d'intervention **99,** et pour d'autre part prélever du liquide chargé de débris dans le site clinique (par la conduite 93) et le diriger vers une évacuation, comme ceci est connu en soi et donc non détaillé plus amplement dans la suite.

Un outil électro-chirurgical comprend un générateur repéré **8** et une pièce à main de type bistouri ou shaver (repère **81),** les deux étant reliés par un câble de commande **1** qui comprend plusieurs conducteurs électriques comme ceci est connu en soi et donc non détaillé plus amplement dans la suite.Sur les figures et dans la description qui suit, les mêmes éléments portent les mêmes signes de référence aux dessins.

On appelle face avant, ou façade, la face de l'appareil destinée à être tournée vers l'utilisateur lors de l'utilisation de l'appareil reposant sur un support. Les termes « avant » et « arrière », « devant » et « derrière », « dessus » et « dessous », « supérieur(e) » et « inférieur(e) », « haut » et « bas », « latéral(e) » et « côté », « droit(e) » et « gauche », notamment, sont utilisés dans la suite en référence à cette convention.

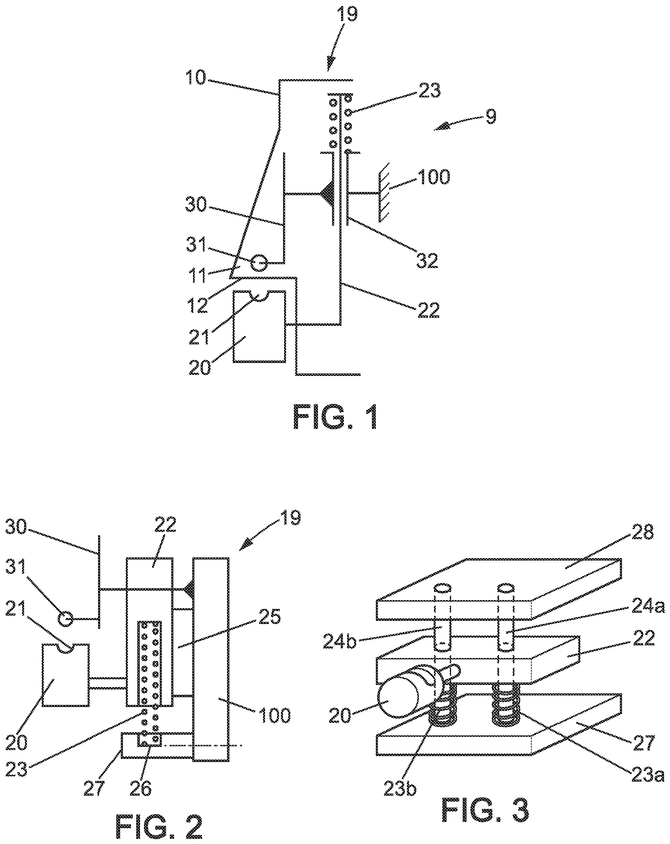

En référence au schéma fonctionnel de la Figure 1, l'extérieur de l'appareil 9 est l'environnement à gauche d'une face **10** déterminée d'un boîtier de l'appareil, par exemple et de préférence la face avant de l'appareil.

Mais il peut aussi s'agir d'une face latérale, d'une face supérieure ou d'une face arrière du boîtier. L'intérieur de l'appareil est l'espace représenté à droite de la face avant **10** du boîtier. Le boîtier de l'appareil comprend un châssis sur lequel la façade **10** vient se fixer, par vissage, encliquetage, ou par tout autre moyen. Sur la Figure 1, le châssis est représenté symboliquement par le symbole de masse électrique.

Le dispositif de maintien de câble, repéré 19, est incorporé dans l'appareil. Il comprend un bouton de préhension **20** accessible par un utilisateur depuis l'extérieur de l'appareil. Dans l'exemple représenté, le dispositif de maintien de câble comprend une partie mobile **22** qui peut coulisser parallèlement au plan de la face avant de l'appareil, lorsque l'utilisateur tire le bouton **20** vers le bas, qui fait partie d'une glissière munie d'un renvoi d'angle. Des moyens de rappel élastique, comme par exemple au moins un ressort **23,** permettent au dispositif de blocage de revenir dans sa position initiale (position de repos) lorsque l'utilisateur relâche le bouton de préhension. Dans d'autres modes de réalisation (non représentés), le dispositif de blocage de câble peut coulisser dans l'autre sens, c'est-à-dire vers le haut en réponse à une action correspondante de l'utilisateur.

Le bouton **20** comprend une échancrure **21,** par exemple de forme semi-circulaire en coupe, du côté de la position de repos du bouton si l'on considère sa course entre deux positions extrêmes résultant des actions contraires de l'utilisateur et des moyens de rappel élastique. Cette échancrure vient au droit d'un plan de contact **12** formé par une portion **11** de la face avant du boîtier venant en saillie par rapport au plan général d'extension de ladite face avant. En variante, le bouton **20** est logé dans un renfoncement de la face avant par rapport au plan général d'extension de la face avant, qui fournit le plan de contact **12** en regard de l'échancrure **21** du bouton. Le diamètre de l'échancrure **21,** et l'espace entre le fond de l'échancrure et le plan de contact **12** sont tels que des câbles d'alimentation électro-médicaux de différentes sections usuelles peuvent être amenés à reposer dans l'échancrure lorsque l'utilisateur tire le bouton vers le bas, et être pincés contre la paroi de contact **12** lorsque l'utilisateur relâche le bouton et que celui-ci remonte sous l'effet du ressort de rappel **22.**

Toujours en référence à la Figure 1, l'appareil comprend en outre une carte électronique **30** avec une antenne **31** associée à un circuit résonnant, pour capter un signal correspondant à un bruit électronique à large bande spectrale généré par la circulation dans le câble d'un courant d'alimentation d'un outil chirurgical électro-médical. L'antenne **31** est par exemple une bobine en composant discret. Il peut aussi s'agir d'une bobine gravée sur un susbtrat telle que le substrat de la carte électronique **30,** ou un autre substrat qui est rapporté sur la carte. La carte électronique **30** peut aussi comprendre, de manière non obligatoire et non limitative, un circuit d'amplification et de traitement du signal capté, pour générer un signal d'activation conditionnelle de la mise en service de la pompe ou de toute autre fonction de l'appareil.

La carte électronique est couplée, mécaniquement et électriquement, à au châssis **100** de l'appareil qui est à la masse électrique, par l'intermédiaire d'une ou plusieurs bride, et/ou par l'intermédiaire d'un étrier **40** ayant deux branches s'étendant perpendiculairement à la face du boîtier considérée.

Dans l'exemple représenté, la glissière **23** du dispositif de blocage de câble passe à travers l'étrier **40.** La carte électronique est agencée à l'intérieur du boîtier de manière que l'antenne **31** qu'elle porte est au droit de l'échancrure **21** du bouton de préhension **20.** Celui-ci permet donc de maintenir le câble de l'outil chirurgical à proximité de l'antenne, c'est-à-dire à une distance comprise entre environ 2 cm et environ 3 cm, par exemple, selon la technologie de l'antenne et les performances du circuit d'amplification et de traitement du signal détecté.

Ainsi équipée et agencée, la carte électronique **30** avec l'antenne **31** forme un dispositif de détection de courant, directement intégré dans l'appareil au niveau de la face considérée du boîtier et au niveau en outre du dispositif de blocage de câble, de manière à pouvoir détecter la circulation d'un courant dans un câble qui est bloqué dans le dispositif de blocage de câble.

La Figure 2 montre, de façon encore schématique, un premier mode d'agencement du dispositif de blocage de câble et du dispositif de détection de courant de l'appareil. Sur cette figure, la façade du boîtier de l'appareil n'est pas représentée. La figure montre un premier mode de réalisation possible des moyens élastiques assurant le retour du dispositif de blocage de câble dans sa position de repos. Dans ce mode de réalisation, la glissière **22** présente un corps mobile ayant la forme générale d'un parallélépipède rectangle. Le corps **22** présente au moins une rainure longitudinale (non visible à la Figure 2) coopérant avec une partie dormante de glissière **25** qui est solidaire de la carcasse **100** de l'appareil et présente une forme en coupe correspondant à celle de la rainure. Le ressort de rappel **23** est agencé dans un évidement longitudinal prévu dans le corps de la glissière **22,** lequel évidement débouche du corps **22** vers le bas de celui-ci. La longueur du ressort **23** est supérieure à la profondeur de l'évidement, de manière que la portion du ressort qui dépasse de l'évidement repose sur un socle **27** qui s'étend à partir de la carcasse **100** dans une direction transversale à l'axe longitudinal du corps **22** et de son évidement. Plus particulièrement, l'extrémité inférieure du ressort, c'est-à-dire celle opposée à l'extrémité du ressort qui vient en appui contre le fond de l'évidement dans le corps **22,** s'engage dans un évidement **26** prévu dans le socle **27** et débouchant de la face supérieure de celui-ci, et dont l'ouverture est en regard de l'ouverture de l'évidement dans le corps **22.** De cette manière, le ressort est stable, et peut travailler en compression lorsque l'utilisateur tire le bouton **20** vers le bas afin d'engager un câble dans l'échancrure **21.**

La Figure 3 illustre un autre mode d'agencement des moyens élastiques de rappel du bouton de préhension **20.** Dans cette forme de réalisation, la partie mobile de la glissière **22** présente également un corps de forme générale parallélépipédique rectangle, mais celui-ci comporte au moins deux évidements cylindriques au lieu d'une ou plusieurs rainures comme dans le mode de réalisation de la Figure 2. Le corps **22** peut coulisser suivant un axe vertical le long de deux poteaux **24a** et **24b** par chacun de ces deux ces évidements, respectivement. Les poteaux **24a** et **24b** sont solidaires d'une platine inférieure **27** et d'une platine supérieur **28,** qui s'étendent transversalement à l'axe longitudinal des poteaux et au plan général de la façade de l'appareil, depuis le châssis de celui-ci (non représenté à la Figure 3, mais correspondant au châssis **100** des Figures 1 et 2). Deux ressorts hélicoïdaux **23a** et **23b** sont disposés autour des poteaux **24a** et **24b,** respectivement, dont ils sont concentriques. Plus particulièrement, les ressorts **23a** et **23b** sont disposés entre le corps mobile **22** de la glissière et la platine inférieure **27** contre lesquels ils sont en appui par chacune de leurs extrémités respectives. Ainsi montés, ils travaillent en compression lorsqu'un utilisateur tire sur le bouton de commande **20** pour engager un câble dans son échancrure de passage de câble. Lorsque l'utilisateur relâche le bouton, celui-ci remonte vers le haut sous l'action des ressorts en sorte que le câble est pincé comme il a été exposé plus haut en référence au schéma cinématique de la Figure 1.

D'autres façons d'agencer les moyens de rappels élastiques sont envisageables et peuvent être choisies en fonction, notamment, de la disposition du bouton de préhension **20** (en façade ou sur une face latérale ou supérieure du boîtier de l'appareil), de sa direction et de son sens de déplacement.

On notera que la direction de déplacement du bouton 20 peut également être différente de la verticale. Elle peut par exemple être horizontale, par exemple orthogonale au plan de la face du boîtier considérée en sorte que l'utilisateur doit tirer le bouton vers lui à la manière d'une tirette pour pouvoir engager le câble, et que le câble est pincé lorsque l'utilisateur relâche la tirette. Elle encore être horizontale et parallèle au plan général de la face considérée du boîtier de l'appareil.

On notera aussi que le bouton de préhension peut être monté pivotant, au lieu d'être monté coulissant comme dans les modes de réalisation représentés.

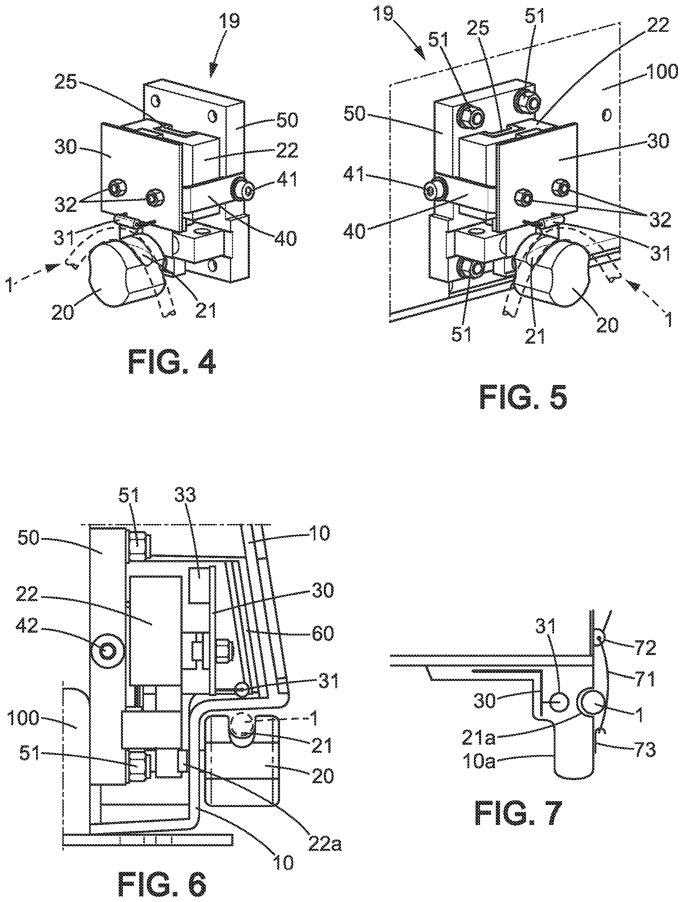

La Figure 4 et la Figure 5 montrent des vues en trois dimensions, respectivement du côté gauche et du côté droit, du dispositif de blocage de câble et du dispositif de détection de courant, de la pompe d'arthroscopie 9 selon un mode de réalisation de l'invention conforme à la Figure 2.

Les différents moyens de ces dispositifs, qui ont été décrits plus haut en référence aux schémas des figures 1 à 3, sont ici montés sur une semelle 50, par exemple une semelle métallique mais qui peut aussi être en polycarbonate si une connexion à la masse électrique du châssis peut être réalisée autrement que par la semelle **50.** Cette semelle **50** est en effet fixée sur le châssis **100** de l'appareil comme montré à la Figure 5, par exemple par des vis **51.**

La carte électronique est fixée à un étrier **40** par des vis **32.** L'étrier **40** est fixé à la semelle **50** par des vis **41.**

La partie mobile **22** de la glissière, du moins la portion de celle-ci qui de déplace verticalement, coulisse dans l'étrier **40,** donc entre la face arrière de la carte électronique **30** (si l'on considère que la face avant de la carte est celle tournée vers l'extérieur du boîtier de l'appareil) et la semelle **50.** La partie dormante **25** de la glissière est solidaire de la semelle **50.**

La Figure 6 est une vue en coupe latérale des dispositifs des Figures 4 et 5, dans un plan de coupe passant par le corps **22** de la glissière. On ne voit donc pas l'étrier **40** de maintien de la carte électronique **30** sur la Figure 6. Le trou **42** correspond au trou taraudé pour recevoir l'une des vis **32** de fixation de cet étrier à la semelle **50.**

A la Figure 6, à l'inverse, on voit un circuit électronique **33,** qui peut comme dans l'exemple représenté être disposé sur la face arrière de la carte électronique **30** (celle tournée vers l'intérieur du boîtier), alors que l'antenne **31** est de préférence agencée sur la face avant de la carte (celle tournée vers l'extérieur du boîtier). Le circuit **33** peut comprendre à un circuit résonnant associé à l'antenne **31** pour capter un signal correspondant à un bruit électronique à large bande spectrale généré par la circulation dans un câble loqué dans l'échancrure **21** d'un courant d'alimentation d'un outil chirurgical électro-médical. Il peut aussi comprendre un circuit d'amplification, et un circuit de traitement du signal capté pour générer un signal d'activation conditionnelle de la mise en service de la fonction de l'appareil, à savoir le pompage dans le cas d'une pompe d'arthroscopie. L'Homme de métier appréciera que ces différents éléments peuvent être réalisés sous la forme de composants électronique discrets, ou d'un ou plusieurs circuits intégrés, ou d'une combinaison des deux.

Le système peut comprendre un blindage **60,** par exemple une feuille métallique au dos de la face interne de la façade **10** du boîtier, au niveau de la carte électronique **30.** Le fait que la carte électronique soit ainsi installée dans un environnement blindé permet d'améliorer l'immunité aux parasites, notamment aux parasites électromagnétiques, et ainsi d'éviter ou de limiter les fausses détections.

Dans certains modes de réalisation, le système médical peut être peut outre comprendre un capteur à effet Hall dont le signal de détection est par exemple exploité par le circuit électronique **33** pour détecter la présence d'un câble. La prise en compte du signal de détection de la présence d'un câble permet aussi d'éviter ou du moins de réduire le risque de fausses alarmes, et donc la mise en service intempestive de la pompe d'arthroscopie.

Enfin la Figure 6 permet de montrer un axe **22a** qui assure le couplage mécanique du bouton de préhension **20** avec la partie mobile **22** de la glissière. Le mouvement vers le bas imprimé par l'utilisateur sur le bouton **20** est transmis à la glissière **22** via cet axe **22a.** L'axe traverse la façade avant **10** de l'appareil, en sorte que seul le bouton est visible et accessible par l'utilisateur depuis l'extérieur du boîtier de l'appareil. On notera que la façade avant **10** du boîtier comprend à cet effet un trou oblong s'étendant suivant la direction de déplacement de l'axe **22a,** c'est à-dire suivant la verticale dans le mode de réalisation représenté.

Par exemple, un mode de réalisation représenté à la Figure 7 ne comprend pas de bouton de préhension dans le dispositif de blocage de câble. Le blocage du câble est assuré par d'autres moyens. Une échancrure **21a** peut être prévue, par exemple, sur un côté latéral d'un élément de façade **10a** du boîtier de l'appareil. Par l'expression « côté latéral », on entend ici un côté de l'élément **10a** venant en alignement et en prolongement avec une face latérale du boîtier de l'appareil (i.e., une face du boîtier perpendiculaire à la façade **10** qui correspond à la face avant du boîtier). Le blocage du câble **1** dans l'échancrure **21a** est assuré, dans l'exemple représenté, par une grenouillère dite aussi « pince grenouille ». Cette grenouillère comprend une boucle **71** qui est solidaire du boîtier tout en étant libre en rotation via un pivot fixé sur la face latérale considérée. Cette boucle peut être engagée dans un crochet **73** fixé à l'élément de façade **10a.** Lorsque la boucle **71** de la grenouillère est tendue et verrouillée, elle appuie sur le câble 1 qui est alors pincé dans l'échancrure 21a.

La carte électronique **30** est fixée au châssis du boîtier de l'appareil, directement ou indirectement, et l'antenne **31** est disposée au plus près de l'échancrure prévue pour recevoir le câble d'alimentation de l'outil chirurgical électro-médical.

On remarque que le câble externe 1 occupe une position de passage (ou 'de transit') par rapport à la face de boîtier 10 de la pompe arthroscopique 9. Il n'y a pas de contact électrique direct entre les conducteurs du câble 1 de l'outil électro-médical (câble 1 dit par conséquent « externe » à juste titre) et l'appareil arthroscopique 9.

On note aussi que la position de passage du câble externe 1 à proximité de la face de boîtier est telle que le câble externe s'étend généralement parallèlement à la face de boîtier à cet endroit ; autrement dit le câble externe s'étend généralement dans une position adjacente à la face de boîtier.

On remarque que l'appareil électro-médical peut comporter une seule pompe ; dans l'exemple illustré il comporte deux pompes. Mais, des configurations à plus de 2 pompes sont également envisageables.

## Revendications

1. **Appareil électro-médical** comprenant un **boîtier** avec un **châssis** et au moins une **face de boîtier** (10) accessible par un utilisateur, et un dispositif interne d'activation conditionnelle de la mise en service d'au moins une fonction de l'appareil en réponse à la détection d'un courant électrique circulant dans un câble externe à l'appareil (1), **caractérisé en ce qu'**il comprend en outre un système ayant :
- un **dispositif de maintien de câble** (19) agencé sur la face de boîtier, du côté externe à l'appareil, pour maintenir ledit câble externe à l'appareil (1) au niveau de la face de boîtier, dans une position de passage à proximité de la face de boîtier, et
- un **dispositif de détection de courant** (30-31), intégré dans l'appareil au niveau de la face de boîtier et du dispositif de maintien de câble, pour détecter la circulation d'un courant dans ledit câble (1) externe à l'appareil qui est maintenu dans le dispositif de maintien de câble.

2. Appareil selon la revendication 1, dans lequel le dispositif de détection de courant comprend une **carte électronique** (30) avec une **antenne** (31) associée à un circuit résonnant pour capter un signal correspondant à un bruit électronique à large bande spectrale généré par la circulation dans le câble d'un courant d'alimentation d'un outil chirurgical électro-médical, et à un circuit d'amplification et de traitement du signal capté pour générer un signal d'activation conditionnelle de la mise en service de la fonction de l'appareil.

3. Appareil selon la revendication 2, dans lequel la carte électronique est fixée au châssis à l'intérieur du boîtier de l'appareil par un **étrier de fixation** (40) de telle manière qu'elle s'étend parallèlement à la face de boîtier (10), et dans lequel le dispositif de maintien de câble comprend une glissière avec une partie mobile (22) agencée pour coulisser entre les branches de l'étrier de fixation parallèlement à la face de boîtier, à l'intérieur du boîtier.

4. Appareil selon la revendication 3, dans lequel la partie mobile de la glissière présente une rainure longitudinale s'étendant suivant la direction de déplacement de la partie mobile de la glissière, et dans lequel la glissière comprend une partie dormante fixée au châssis.

5. Appareil selon l'une quelconque des revendications 3 et 4, dans lequel le dispositif de maintien de câble comprend en outre un **bouton de préhension** (20) accessible par un utilisateur depuis l'extérieur de l'appareil et couplé mécaniquement à la partie mobile (22) de la glissière à l'intérieur du boîtier, à travers la face de boîtier, ainsi que des moyens de rappel élastique pour ramener le bouton de préhension dans une position de repos lorsque l'utilisateur relâche le bouton de préhension après avoir exercé une action tendant à le déplacer par rapport à ladite position de repos.

6. Appareil selon la revendication 5, dans lequel le bouton de préhension (20) comprend une **échancrure** (21) du côté de la position de repos du bouton si l'on considère la course dudit bouton entre deux positions extrêmes résultant des actions contraires de l'utilisateur et des moyens de rappel élastiques, pour recevoir le câble externe à l'appareil.

7. Appareil selon l'une quelconque des revendications 5 et 6, dans lequel la face de boîtier un plan de contact (12) en regard de l'échancrure (21) du bouton de préhension, le diamètre de l'échancrure et l'espace entre le fond de l'échancrure et le plan de contact étant tels qu'un câble externe à l'appareil peut être amené à reposer dans l'échancrure lorsque l'utilisateur exerce une action sur le bouton tendant à la déplacer par rapport à sa position de repos et est pincé contre la paroi de contact lorsque l'utilisateur relâche le bouton et que le bouton revient dans sa position de repos sous l'effet des moyens de rappel élastiques.

8. Appareil selon l'une quelconque des revendications précédentes, dans lequel la face de boîtier comprend en outre un **blindage** (60) de protection électromagnétique de la carte électronique (30) du côté de la face de boîtier, au niveau de la carte électronique (30).

9. Appareil selon l'une quelconque des revendications précédentes, dans lequel le système comprend en outre un **capteur à effet Hall** pour détecter la présence d'un câble bloqué dans le dispositif de maintien de câble.

10. Appareil selon l'une quelconque des revendications précédentes constituant une **pompe d'arthroscopie** dans laquelle la fonction mise en service en réponse à la détection d'un courant électrique circulant dans le câble externe à l'appareil comprend l'irrigation par un fluide propre et l'aspiration de fluides souillés au niveau d'un site d'intervention chirurgicale pratiquée à l'aide d'outils chirurgicaux électro-médicaux alimentés électriquement par le câble externe à l'appareil.

11. Appareil selon l'une des revendications 1 à 9, comportant au moins une pompe.

12. Appareil selon l'une des revendications précédentes, dans lequel la position de passage du câble externe (1) à proximité de la face de boîtier est telle que le câble externe s'étend généralement parallèlement à la face de boîtiers à cet endroit.

## Patentansprüche

1. **Elektromedizinische Vorrichtung,** umfassend ein **Gehäuse** mit einem **Rahmen** und wenigstens einer **Gehäusefläche** (10), die für einen Benutzer zugänglich ist, und einer internen Vorrichtung zur bedingten Aktivierung der Inbetriebnahme wenigstens einer Funktion der Vorrichtung als Reaktion auf die Erfassung eines elektrischen Stroms, der in einem Kabel außerhalb der Vorrichtung (1) fließt, **dadurch gekennzeichnet, dass** es ferner ein System umfasst, das Folgendes aufweist:
- eine **Kabelhaltevorrichtung** (19), die an der Gehäusefläche auf der Außenseite der Vorrichtung angeordnet ist, um das Kabel außerhalb der Vorrichtung (1) an der Gehäusefläche in einer Durchgangsposition in der Nähe der Gehäusefläche zu halten; und
- eine **Stromerfassungsvorrichtung** (30-31), die an der Gehäusefläche und der Kabelhaltevorrichtung in die Vorrichtung integriert ist, um den Stromfluss in dem Kabel (1) außerhalb der Vorrichtung, die in der Kabelhaltevorrichtung gehalten ist, zu erfassen.

2. Vorrichtung nach Anspruch 1, wobei die Stromerfassungsvorrichtung eine **elektronische Karte** (30) mit einer **Antenne** (31) umfasst, die mit einem Resonanzkreis verbunden ist, um ein Signal zu erfassen, das einem elektronischen Rauschen mit breitem Spektralbereich entspricht, das durch den Fluss eines Versorgungsstroms eines elektromedizinischen chirurgischen Werkzeugs in dem Kabel erzeugt wird, und mit einem Schaltkreis zur Verstärkung und Verarbeitung des erfassten Signals, um ein Signal zur bedingten Aktivierung der Inbetriebnahme der Funktion der Vorrichtung zu erzeugen.

3. Vorrichtung nach Anspruch 2, wobei die elektronische Karte im Inneren des Gehäuses der Vorrichtung durch einen **Befestigungsbügel** (40) derart an dem Rahmen befestigt ist, dass sie sich parallel zur Gehäusefläche (10) erstreckt, und wobei die Kabelhaltevorrichtung eine Gleitschiene mit einem beweglichen Teil (22) umfasst, der so angeordnet ist, dass er zwischen den Schenkeln des Befestigungsbügels parallel zur Gehäusefläche im Inneren des Gehäuses gleitet.

4. Vorrichtung nach Anspruch 3, wobei der bewegliche Teil der Gleitschiene eine Längsnut aufweist, die sich in der Bewegungsrichtung des beweglichen Teils der Gleitschiene erstreckt, und wobei die Gleitschiene einen am Rahmen befestigten Rahmenteil umfasst.

5. Vorrichtung nach einem der Ansprüche 3 oder 4, wobei die Kabelhaltevorrichtung ferner einen **Griffknopf** (20) umfasst, der für einen Benutzer von außerhalb der Vorrichtung zugänglich ist und mechanisch mit dem beweglichen Teil (22) der Gleitschiene im Inneren des Gehäuses durch die Gehäusefläche hindurch gekoppelt ist, sowie elastische Rückstellmittel, um den Griffknopf in eine Ruhestellung zurückzuführen, wenn der Benutzer den Griffknopf loslässt, nachdem er eine Aktion ausgeführt hat, die darauf abzielt, den Knopf aus der Ruhestellung heraus zu bewegen.

6. Vorrichtung nach Anspruch 5, wobei der Griffknopf (20) eine **Aussparung** (21) auf der Seite der Ruheposition des Knopfes aufweist, wenn man die Bewegung des Knopfes zwischen zwei Endpositionen betrachtet, die aus den entgegengesetzten Aktionen des Benutzers und den elastischen Rückstellmitteln resultiert, um das Kabel außerhalb der Vorrichtung aufzunehmen.

7. Vorrichtung nach einem der Ansprüche 5 und 6, wobei die Gehäusefläche eine Kontaktebene (12) aufweist, die der **Aussparung** (21) des Griffknopfes gegenüberliegt, wobei der Durchmesser der Aussparung und der Raum zwischen dem Boden der Aussparung und der Kontaktebene derart beschaffen sind, dass ein Kabel außerhalb der Vorrichtung in der Aussparung zu liegen kommen kann, wenn der Benutzer eine Aktion ausführt, die darauf abzielt, den Knopf aus seiner Ruhestellung heraus zu bewegen, und das Kabel gegen die Kontaktwand gedrückt wird, wenn der Benutzer den Knopf loslässt und der Knopf unter der Wirkung der elastischen Rückstellmittel in seine Ruhestellung zurückkehrt.

8. Vorrichtung nach einem der vorhergehenden Ansprüche, wobei die Gehäusefläche ferner eine **Abschirmung** (60) zum elektromagnetischen Schutz der elektronischen Karte (30) auf der Seite der Gehäusefläche in Höhe der elektronischen Karte (30) umfasst.

9. Vorrichtung nach einem der vorhergehenden Ansprüche, wobei das System ferner einen **Hall-Effekt-Sensor** zur Erkennung eines in der Kabelhaltevorrichtung blockierten Kabels umfasst.

10. Vorrichtung nach einem der vorhergehenden Ansprüche, welches eine **Arthroskopiepumpe** bildet, wobei die Funktion, die als Reaktion auf die Erfassung eines elektrischen Stroms, der durch das externe Kabel der Vorrichtung fließt, aktiviert wird, das Spülen mit sauberer Flüssigkeit und das Absaugen von verschmutzten Flüssigkeiten an einer Stelle eines chirurgischen Eingriffs umfasst, der mit Hilfe von elektromedizinischen chirurgischen Werkzeugen durchgeführt wird, die durch das externe Kabel der Vorrichtung mit Strom versorgt werden.

11. Vorrichtung nach einem der Ansprüche 1 bis 9, umfassend wenigstens eine Pumpe.

12. Vorrichtung nach einem der vorhergehenden Ansprüche, wobei die Position des Durchgangs des externen Kabels (1) in der Nähe der Gehäusefläche derart ist, dass sich das externe Kabel an dieser Stelle im Allgemeinen parallel zur Gehäusefläche erstreckt.

## Claims

1. Electro-medical appliance comprising a housing with a frame and at least one housing face (10) accessible by a user, and an internal device for the conditional activation of at least one function of the appliance in response to the detection of an electric current circulating in a cable outside the appliance (1), **characterised in that** it further comprises a system having:
- a cable-holding device (19) arranged on the housing face, on the outer side of the appliance, for holding said cable outside the appliance (1) in the region of the housing face, in a position passing close to the housing face, and
- a current detecting device (30-31) built into the appliance in the region of the housing face and the cable-holding device, for detecting the circulation of current in said cable (1) outside the appliance which is held in the cable-holding device.

2. Appliance according to claim 1, wherein the device for detecting current comprises an electronic card (30) with an antenna (31) associated with a resonant circuit for sensing a signal corresponding to an electronic noise with a broad spectrum bandwidth generated by the circulation in the cable of a power supply current for an electro-medical surgical tool, and a circuit for amplifying and processing the signal received for generating a signal for the conditional activation of the function of the appliance.

3. Appliance according to claim 2, wherein the electronic card is fixed to the frame inside the housing of the appliance by a fixing bracket (40) in such a way that it extends parallel to the housing face (10), and wherein the cable-holding device comprises a slide with a movable part (22) arranged to slide between the legs of the fixing bracket parallel to the housing face, inside the housing.

4. Appliance according to claim 3, wherein the movable part of the slide has a longitudinal groove extending in the direction of movement of the movable part of the slide, and wherein the slide comprises a stationary part fixed to the frame.

5. Appliance according to any of claims 3 and 4, wherein the cable-holding device further comprises a gripping knob (20) accessible by user from the outside of the appliance and coupled mechanically to the movable part (22) of the slide inside the housing, through the housing face, as well as resilient return means for returning the gripping knob to a rest position when the user releases the gripping knob after having exerted an action that displaces it relative to said rest position.

6. Appliance according to claim 5, wherein the gripping knob (20) comprises an indentation (21) on the resting side position of the knob, considering that the path of said knob is between two extreme positions resulting from opposite actions of the user and the elastic return means, for receiving the cable outside the appliance.

7. Appliance according to any of claims 5 and 6, wherein the housing face a plane of contact (12) facing the indentation (21) of the gripping knob, the diameter of the indentation and the space between the bottom of the indentation and the plane of contact being such that a cable outside the appliance can be brought to lie in the indentation when the user exerts an action on the knob aiming to move it from its rest position and is pinched against the contact wall when the user releases the knob and the knob returns to its rest position due the effect of the resilient return means.

8. Appliance according to any of the preceding claims, wherein the housing face further comprises a shield (60) for the electromagnetic protection of the electronic card (30) on the housing face side in the region of the electronic card (30).

9. Appliance according to any of the preceding claims, wherein the system further comprises a Hall effect sensor for detecting the presence of a jammed cable in the cable-holding device.

10. Appliance according to any of the preceding claims constituting an arthroscopy pump in which the function activated in response to the detection of an electric current circulating through the cable outside the appliance comprises irrigation with clean fluid and the aspiration of contaminated fluids from a surgical intervention site formed by electro-medical surgical tools that are powered electrically by the cable outside the appliance.

11. Appliance according to any of claims 1 to 9, including at least one pump.

12. Appliance according to any of the preceding claims, wherein the passing position of the outer cable (1) close to the housing face is such that the outer cable extends generally parallel to the housing face at this point.
